## Europäisches Patentamt
## European Patent Office
## Office européen des brevets

(19)

(11) Numéro de publication: **0 015 831**
**B1**

(12) **FASCICULE DE BREVET EUROPEEN**

(45) Date de publication du fascicule de brevet: **30.12.81**

(21) Numéro de dépôt: **80400278.0**

(22) Date de dépôt: **29.02.80**

(51) Int. Cl.³: **C 07 C 7/00, C 07 C 7/148, C 07 C 15/24, C 07 C 147/06**

(54) **Procédé de préparation de naphtalène sulfoné au moins en partie et application du produit obtenu à la purification du naphtalène.**

(30) Priorité: **02.03.79 FR 7905433**

(43) Date de publication de la demande:
**17.09.80 Bulletin 80/19**

(45) Mention de la délivrance du brevet:
**30.12.81 Bulletin 81/52**

(84) Etats Contractants Désignés:
**AT BE DE GB IT NL**

(56) Documents cités:
**FR - A - 1 300 453**
**FR - A - 1 349 806**
**FR - A - 1 600 601**
**FR - A - 2 277 061**
**GB - A - 760 054**
**GB - A - 1 371 232**
**US - A - 2 955 144**

**CHEMICAL ABSTRACTS, vol. 71, 1969 réf. 61059p, page 414**
**Columbus, Ohio, US**

**DERWENT JAPANESE PATENTS REPORT, vol. 74, no. 37, 17—10—1974**
**Derwent Publications Londres GB**

(73) Titulaire: **Société Chimique des Charbonnages**
**Tour Aurore Place des Reflets Cédex no 5**
**F-92080 Paris La Défense 2 (FR)**

(72) Inventeur: **Calin, Paul**
**10, rue Jean Létienne**
**F-62300 Lens (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de naphtalène sulfoné au moins en partie et application du produit obtenu à la purification du naphtalène

La présente invention concerne la sulfonation au moins partielle, du naphtalène et l'application du produit obtenu à la purification chimique du naphtalène par réaction avec du formaldéhyde.

Le naphtalène contient souvent, même s'il est obtenu par distillation, des quantités non négligeables de produits soufrés qui sont des poisons des catalyseurs utilisés, en particulier, lors de l'oxydation du naphtalène en anhydride phtalique, ou de son hydrogénation en tétraline et décaline.

On connait déjà des procédés chimiques permettant de diminuer la teneur en impuretés soufrées du naphtalène. Ainsi le brevet anglais n° 760.054 décrit un procédé consistant à chauffer le naphtalène liquide en présence d'acide sulfurique et de paraformaldéhyde puis à neutraliser le milieu réactionnel et distiller le naphtalène purifié. Le brevet américain n° 2.955.144 décrit un procédé consistant à laver à chaud le naphtalène par l'acides sulfurique, à séparer la phase aqueuse, puis à continuer le traitement du naphtalène en présence d'une solution aqueuse de formaldéhyde. Le brevet anglais n° 1.371.232 décrit un procédé dans lequel on traite d'abord le naphtalène par l'acide sulfurique puis on élimine l'acide dilué en excès, on poursuit le traitement à l'aide de paraformaldéhyde ou de formaldéhyde et on distille le naphtalène. Le brevet de l'Allemagne de l'Ouest n° 2.432.066 décrit un procédé consistant à ajouter au naphtalène impur, à chaud, un acide sulfonique puis du paraformaldéhyde, à séparer la phase aqueuse, neutraliser la phase organique et distiller le naphtalène purifié.

Les procédés selon l'art antérieur ne donnent pas en tous points satisfaction: soit qu'ils conduisent au rejet d'eaux usées polluantes, soit qu'ils nécessitent des équipements spécialement résistants à la corrosion (acier émaillé ou vitrifié), soit (cas du traitement par les acides sulfoniques purs) qu'ils nécessitent des temps de réaction trop longs.

La demanderesse à maintenant trouvé que la purification du naphtalène contenant des impuretés soufrées, de manière à réduire la teneur finale en soufre à moins de 1000 ppm, est possible en évitant le rejet d'eaux usées polluantes, en évitant l'utilisation de matériels spéciaux et/ou très sophistiqués, et en opérant en des temps plus courts, à condition d'effecteur le traitement à l'acide sulfurique et au formaldéhyde en deux étapes réactionnelles nettement distinctes, la première étant une sulfonation au moins partielle par l'acide sulfurique d'un naphtalène à purifier dans des conditions telles que tout l'acide sulfurique réagisse et que le produit obtenu soit exempt d'eau, la seconde étant un traitement par le formaldéhyde du produit de la réaction de sulfonation, en présence, le cas échéant, d'un ajout de naphtalène à purifier.

Il est connu de réaliser la sulfonation d'un hydrocarbure aromatique à l'aide d'acide sulfurique en présence d'un solvant chargé d'éliminer l'eau formée, par azéotropie. Ainsi le brevet polonais n° 56.590 (résumé dans Chemical Abstracts, Volume 71 (1969) n° 61059p) décrit la sulfonation du naphtalène par l'acide sulfurique à 160°C en présence d'un mélange d'hydrocarbures aliphatiques. La quantité d'acide sulfurique utilisée est supérieure à celle nécessaire pour obtenir l'acide naphtalène-monosulfonique. Le brevet japonais n° 74/033.192, résumé dans Derwent Japanese Patents Report, volume 74, n° 37 du 17/10/1974, enseigne la sulfonation du méta-xylène par un excès d'acide sulfurique à 150—190°C, l'eau formée étant enlevée du milieu réactionnel par distillation azéotropique.

On a maintenant trouvé que la sulfonation du naphtalène est possible en utilisant un défaut d'acide sulfurique (par rapport à la quantité d'acide théoriquement nécessaire pour former l'acide naphtalène monosulfonique) et une température inférieure à 150°C, en éliminant l'eau formée par azéotropie, de telle façon que le produit obtenu, soit totalement exempt d'eau et d'acide sulfurique.

Ainsi un premier objet de la présente invention est un procédé de préparation de naphtalène sulfoné au moins en partie par réaction d'acide sulfurique sur du naphtalène, en présence d'un solvant formant un azéotrope avec l'eau, caractérisé en ce que le rapport molaire

$$\frac{\text{acide sulfurique}}{\text{naphtalène}}$$

est inférieur à 1 et en ce que la réaction de l'acide sulfurique sur le naphtalène est réalisée entre 90 et 150°C en éliminant totalement l'eau de façon continue du milieu réactionnel par distillation azéotropique au moyen d'un solvant choisi parmi les hydrocarbures aliphatiques saturés, les hydrocarbures cycloaliphatiques saturés ou leurs mélanges de façon à ce que le produit obtenu soit totalement exempt d'eau et d'acide sulfurique.

Le naphtalène utilisé peut être de toute provenance. En particulier le naphtalène produit lors de la distillation du goudron de houille convient, en ayant été ou non, au préalable, traité en vue d'éliminer les phénols et bases pyridiques qu'il contient.

L'hydrocarbure aliphatique saturé ou cycloaliphatique saturé ou les mélanges de tels hydrocarbures sont choisis parmi les composés suivants: hexane, heptane, octane, isooctane, nonane, cyclohexane, méthylcyclohexane, fractions d'hydrocarbures saturés provenant de la distillation du pétrole. Le choix précis d'un hydrocarbure ou d'un mélange d'hydrocarbures est fonction de la température choisie pour la sulfonation.

Pour une température de sulfonation choisie aux environs de 120 à 130°C, on utilise par exemple l'heptane à raison de 10 à 15 parties en poids pour 100 parties de mélange réactionnel. Dans ces conditions, la sulfonation du naphtalène par la moitié de la quantité théorique d'acide sulfurique nécessaire pour conduire à l'acide naphtalènemonosulfonique demande un temps de 5 à 10 heures (d'autant plus long que la capacité du réacteur est plus importante) pendant lequel l'azéotrope heptane-eau distille, le distillat est condensé puis séparé dans un décanteur et l'heptane recyclé dans le réacteur. La sulfonation est terminée quand il ne se sépare plus d'eau dans le décanteur.

L'enlèvement continu de l'eau (eau de rejet non polluante comprenant l'eau présente au départ et celle qui se forme pendant la réaction) du milieu réactionnel pendant la sulfonation permet d'utiliser des matériaux classiques (acier inoxydable ordinaire au Nickel-Chrome) et également de l'acide sulfurique pouvant contenir jusqu'à 20% en poids d'eau (dans ce cas, l'acide sera ajouté graduellement au mélange de naphtalène et d'hydrocarbure porté à la température de réaction choisie, de façon à éliminer immédiatement l'eau introduite, par distillation azéotropique).

On peut donc utiliser de l'acide sulfurique contenant de 0 à 20% d'eau en poids. Le rapport molaire

$$\frac{\text{acide sulfurique}}{\text{naphtalène}}$$

est inférieur à 1. Il peut même, si on le souhaite, être très inférieur à 1 et correspondre à des quantités d'acide sulfurique concentré (98% en poids) comprises entre 0,5 et parties en poids pour 100 parties en poids de naphtalène.

Le mélange réactionnel, après sulfonation, contient, au moins en partie, l'hydrocarbure ayant servi à éliminer l'eau par distillation azéotropique. Cet hydrocarbure peut être distillé, éventuellement sous pression réduite, et utilisé dans une opération ultérieure. Mais on a constaté que l'application du produit obtenu à la purification du naphtalène ne nécessitait pas l'élimination de l'hydrocarbure.

Un second objet de la présente invention est l'application du naphtalène sulfoné au moins en partie, exempt d'eau et d'acide sulfurique, obtenu selon le procédé décrit ci-dessus, à la purification du naphtalène, caractérisé en ce que:

a) le cas échéant, on mélange le naphtalène sulfoné au moins en partie à du naphtalène à purifier,

b) on fait réagir du formaldéhyde avec le naphtalène sulfoné au moins en partie additionné, le cas échéant, de naphtalène à purifier,

c) on distille le naphtalène contenu dans le milieu réactionnel.

Le naphtalène engagé dans la réaction de sulfonation et le naphtalène ajouté le cas échéant dans la réaction avec le formaldéhyde sont de même provenance ou de provenances différentes. De même que pour la réaction de sulfonation, le naphtalène ajouté, le cas échéant, au naphtalène sulfoné au moins en partie, peut provenir de la distillation du goudron de houille et avoir été débarrassé ou non des phénols et bases pyridiques qu'il contient.

L'ajout de naphtalène à purifier au naphtalène sulfoné au moins en partie est fonction du rapport molaire

$$\frac{\text{acide sulfurique}}{\text{naphtalène}}$$

utilisé pour la sulfonation et est calculé en fonction de la remarque suivante. On a constaté que des quantités d'acide sulfurique concentré comprises entre 0,5 et 2 parties en poids et de préférence voisines de 1 partie en poids, pour 100 parties en poids de naphtalène total à purifier, suffisaient pour réduire, après la réaction avec le formaldéhyde, et distillation, la teneur en soufre du naphtalène distillé à moins de 1000 ppm (0,1% en poids). Ainsi si la réaction de sulfonation est mise en oeuvre avec des quantités d'acide sulfurique concentré comprises entre 0,5 et 2 parties en poids pour 100 parties en poids de naphtalène à purifier, il n'y a pas lieu, avant réaction avec le formaldéhyde, d'ajouter, au naphtalène sulfoné en partie obtenu, du naphtalène à purifier. On ajoute du naphtalène à purifier quand la réaction de sulfonation a été réalisée avec des quantités d'acide sulfurique supérieures à 2 parties en poids pour 100 parties en poids de naphtalène et en particulier avec des quantités comprises entre 15,6 et 47 parties en poids d'acide sulfurique concentré pour 100 parties en poids de naphtalène, qui correspondent à des rapports molaires

$$\frac{\text{acide sulfurique}}{\text{naphtalène}}$$

compris entre 0,2 et 0,6, et qui sont préférées selon l'invention car elles permettent une meilleure agitation du milieu réactionnel, par rapport à des quantités beaucoup plus faibles. Cet ajout sera tel que la quantité globale de naphtalène (engagé d'abord dans la sulfonation plus celui qui est ajouté ensuite avant la réaction avec le formaldéhyde) soit équivalente à celle qui aurait dû être engagée dans la sulfonation si cette réaction avait été réalisée à l'aide de 0,5 à 2 parties en poids d'acide sulfurique concentré pour 100 parties de naphtalène.

Par exemple si la réaction de sulfonation est réalisée en mettant en jeu un rapport molaire

$$\frac{H_2SO_4}{naphtalène} = 0,5,$$

c,est-à-dire 39 parties en poids d'acide sulfurique à 98% en poids, pour 100 parties en poids de naphtalène, on ajoutera, avant traitement par le formaldéhyde, au naphtalène sulfoné en partie obtenu, une quantité en poids de naphtalène à purifier comprise entre 1850 et 7700 fois la quantité en poids de naphtalène engagé dans la réaction de sulfonation.

La formaldéhyde peut être utilisé à l'état gazeux ou en solution aqueuse ou hydro-alcoolique. On préfère l'utiliser à l'état de paraformaldéhyde. La température utilisée pour la réaction du formaldéhyde sera telle que le temps de réaction soit le plus court possible mais elle sera inférieure à la température de dégradation thermique du milieu réactionnel.

On préfère des températures comprises entre 90 et 150°C.

Dans le cas où le produit de la sulfonation au moins partielle du naphtalène est utilisé sans distillation préalable de l'hydrocarbure ayant servi à éliminer l'eau par distillation azéotropique, on pourra également, dans l'opération de purification, éliminer l'eau qui se forme par distillation azéotropique.

L'abaissement de la teneur en soufre du naphtalène est essentiellement fonction de la quantité de formaldéhyde utilisée. Ainsi il est possible, pour une teneur en soufre initiale donnée, d'obtenir les teneurs finales souhaitées en utilisant plus ou moins de formaldéhyde.

On utilise préférentiellement le paraformaldéhyde à des teneurs comprises entre 0,5 et 5 parties en poids pour 100 parties de naphtalène à purifier (comprenant le naphtalène engagé dans la réaction de sulfonation).

Les temps de réaction nécessaires sont de l'ordre de 1 à 3 heures.

Après ce traitement, le milieu réactionnel est, sans neutralisation ni lavage préalables, soumis à une distillation pour éliminer d'abord l'hydrocarbure éventuellement présent et recueillir ensuite le naphtalène purifié.

Le seul sous-produit du procédé selon l'invention (mis à part les résidus de distillation que l'on trouve dans tous les procédés de traitement du naphtalène) est donc de l'eau distillée lors de la sulfonation.

Les exemples non limitatifs suivants sont donnés à titre d'illustration de l'invention.

On a utilisé comme matière première du naphtalène provenant de la distillation du goudron de houille et ayant les caractéristiques suivantes:

Point de cristallisation : 78,6°C
Teneur en soufre : 0,4% en poids
(4 000 ppm)

## Exemple 1

Dans un réacteur muni de moyens de chauffage, d'agitation et de décantation des condensats, on introduit:

Naphtalène .250 kg
Acide sulfurique à 94% en poids 52 kg
Heptane 37,5 kg

Après réaction de 9 h à 125—127°C, pendant laquelle on a éliminé l'eau par distillation azéotropique, on distille l'heptane sous 300 mm de mercure. On obtient ainsi 284,5 kg de naphtalène sulfoné en partie, qui ne contient ni eau ni acide sulfurique.

On introduit dans un réacteur mini de moyens de chauffage et d'agitation:

Naphtalène 400 kg

Naphtalène sulfoné en partie, obtenu ci-dessus 23,2 kg que l'on chauffe à 130°C pendant 0,5 heure. On ajoute ensuite 4 kg de paraformaldéhyde et l'on maintient la température à 130°C pendant 2 h.

La distillation sous pression réduite fournit du naphtalène ayant un point de cristallisation de 79,9°C et une teneur en soufre de 0,095% (950 ppm).

## Exemple 2

On répète l'exemple 1 en utilisant 16 kg de paraformaldéhyde.

Après réaction pendant 1,5 h à 130°C et distillation, on recueille du naphtalène ayant un point de cristallisation de 80°C et une teneur en soufre de 0,0003% (3 ppm).

## Revendications

1. Procédé de préparation de naphtalène sulfoné au moins en partie par réaction d'acide sulfurique sur du naphtalène, en présence d'un solvant formant un azéotrope avec l'eau, caractérisé en ce que le rapport molaire.

$$\frac{acide\ sulfurique}{naphtalène}$$

est inférieur à 1 et en ce que la réaction de l'acide sulfurique sur le naphtalène est réalisée entre 90 et 150°C en éliminant totalement l'eau de façon continue du milieu réactionnel par distillation azéotropique au moyen d'un solvant choisi parmi les hydrocarbures aliphatiques saturés, les hydrocarbures cycloaliphatiques saturés ou leurs mélanges de façon à ce que le produit obtenu soit totalement exempt d'eau et d'acide sulfurique.

2. Procédé selon la revendication 1, caractérisé en ce que le rapport molaire

$$\frac{\text{acide sulfurique}}{\text{naphtalène}}$$

est compris entre 0,2 et 0,6.

3. Application du naphtalène sulfoné au moins en partie, exempt d'eau et d'acide sulfurique, obtenu suivant le procédé de l'une des revendications 1 ou 2, à la purification du naphtalène, caractérisée en ce que:

a) le cas échéant, on mélange le naphtalène sulfoné au moins en partie à du naphtalène à purifier,

b) on fait réagir du formaldéhyde avec le naphtalène sulfoné au moins en partie, additionné, le cas échéant, de naphtalène à purifier,

c) on distille le naphtalène contenu dans le milieu réactionnel.

4. Application selon la revendication 3, caractérisée en ce que le formaldéhyde est utilisé sous la forme de paraformaldéhyde.

5. Application selon la revendication 4, caractérisée en ce que l'on utilise entre 0,5 et 5% en poids de paraformaldéhyde par rapport au naphtalène total à purifier.

6. Application selon l'une des revendications 3 à 5, caractérisée en ce que le naphtalène sulfoné au moins en partie, exempt d'eau et d'acide sulfurique, contient encore au moins partiellement le solvant ayant servi à la distillation azéotropique de l'eau.

7. Application selon la revendication 6, caractérisée en ce que l'eau se formant pendant la phase de traitement à l'aide de formaldéhyde ou de paraformaldéhyde est éliminée azéotropiquement à l'aide du solvant présent.

## Claims

1. Process for the preparation of at least partially sulphonated naphthalene by the reaction of sulphuric acid with naphthalene, in the presence of a solvent forming an azeotrope with water, characterised in that the molar ratio

$$\frac{\text{sulphuric acid}}{\text{naphthalene}}$$

is less than 1 and in that the reaction of the sulphuric acid on the naphthalene is carried out between 90 and 150°C thus totally eliminating water in a continuous manner from the reaction medium by azeotropic distillation by means of a solvent chosen from saturated aliphatic hydrocarbons, saturated cycloaliphatic hydrocarbons or their mixtures so that the product obtained is completely free from water and sulphuric acid.

2. Process according to claim 1, characterised in that the molar ratio

$$\frac{\text{sulphuric acid}}{\text{naphthalene}}$$

is between 0.2 and 0.6.

3. Application of at least partially sulphonated naphthalene, which is free from water and sulphuric acid, obtained according to the process of one of claims 1 or 2, for the purification of naphthalene, characterised in that:

a) if necessary the at least partially sulphonated naphthalene is mixed with the naphthalene to be purified,

b) formaldehyde is reacted with the at least partially sulphonated naphthalene, to which naphthalene to be purified is added, if necessary,

c) the naphthalene contained in the reaction medium is distilled.

4. Application according to claim 3, characterised in that formaldehyde is used in the form of paraformaldehyde.

5. Application according to claim 4, characterised in that between 0.5 and 5% by weight paraformaldehyde is used with respect to the total naphthalene to be purified.

6. Application according to one of claims 3 to 5, characterised in that the at least partially sulphonated naphthalene, free from water and sulphuric acid, still contains at least partially the solvent which served for the azeotropic distillation of water.

7. Application according to claim 6, characterised in that the water forming during the stage of treatment with formaldehyde or paraformaldehyde is eliminated azeotropically with the solvent present.

## Patentansprüche

1. Verfahren zur Herstellung von mindestens teilweise sulfoniertem Naphthalin durch Umsetzung von Naphthalin mit Schwefelsäure in Gegenwart eines ein Azeotrop mit Wasser bildenden Lösungsmittels, dadurch gekennzeichnet, daß ein Molverhältnis Schwefelsäure/Naphthalin <1 angewandt und die Umsetzung des Naphthalins mit Schwefelsäure bei einer Temperatur von 90 bis 150°C durchgeführt wird, wobei das Wasser kontinuierlich durch Azeotrop-Destillation mit einem unter den gesättigten aliphatischen Kohlenwasserstoffen, des gesättigten cycloaliphatischen Kohlenwasserstoffen und ihren Gemischen ausgewählten Lösungsmittel in der Weise vollständig aus dem Reaktionsmedium abgetrennt wird, daß das erhaltene Produkt vollständig frei von Wasser und Schwefelsäure ist.

2. Verfahren nach Anspruch 1, dadurch gekennzeichnet, daß ein Molverhältnis Schwefelsäure/Naphthalin von 0,2 bis 0,6 angewandt wird.

3. Verwendung des nach dem Verfahren von Anspruch 1 oder 2 erhaltenen, mindestens teilweise sulfonierten und wassersowie schwefelsäurefreien Naphthalins zur Reinigung von Naphthalin, gekennzeichnet durch

a) erforderlichenfalls Mischen des mindestens teilweise sulfonierten Naphthalins mit zu reinigendem Naphthalin,

b) Umsetzung von Formaldehyd mit dem mindestens teilweise sulfonierten Naphthalin, das erforderlichenfalls mit zu reinigendem Naphthalin versetzt ist, und

c) Destillation des im Reactionsmedium erhaltenen Naphthalins.

4. Verwendung nach Anspruch 3, dadurch gekennzeichnet, daß der Formaldehyd in Form von Paraformaldehyd eingesetzt wird.

5. Verwendung nach Anspruch 4, dadurch gekennzeichnet, daß 0,5 bis 5 Gew.—% Paraformaldehyd, bezogen auf das Gesamtgewicht des zu reinigenden Naphthalins, eingesetzt werden.

6. Verwendung nach einem der Ansprüche 3 bis 5, dadurch gekennzeichnet, daß ein mindestens teilweise sulfoniertes, wasser- und schwefelsäurefreies Naphthalin verwendet wird, das noch mindestens teilweise das zur Azeotrop-Destillation des Wassers verwendete Lösungsmittel enthält.

7. Verwendung nach Anspruch 6, dadurch gekennzeichnet, daß das sich bei der Behandlung mit Formaldehyd oder Paraformaldehyd bildende Wasser mit dem vorliegenden Lösungsmittel azeotrop abgetrennt wird.